# EUROPEAN PATENT SPECIFICATION

(11) **EP 4 093 464 B1**
(45) Date of publication and mention of the grant of the patent: **23.07.2025**
(21) Application number: 20812098.0
(22) Date of filing: 01.12.2020
(51) Int. Cl.: A61M 5/24, A61M 5/315, A61M 5/32, A61M 5/34, A61M 5/50

(54) **CONTAINER HOLDER ASSEMBLY FOR MEDICAMENT DELIVERY DEVICE, AND MEDICAMENT DELIVERY DEVICE**
BEHÄLTERHALTERANORDNUNG FÜR MEDIKAMENTENVERABREICHUNGSVORRICHTUNG SOWIE MEDIKAMENTENABGABEVORRICHTUNG
ENSEMBLE DE SUPPORT DE RÉCIPIENT POUR DISPOSITIF D'ADMINISTRATION DE MÉDICAMENTS ET DISPOSITIF D'ADMINISTRATION DE MÉDICAMENTS

(30) Priority: 24.01.2020 US 202062965175 P; 30.03.2020 EP 20166553
(43) Date of publication of application: 30.11.2022
(73) Proprietor: SHL Medical AG, 6302 Zug (CH)
(72) Inventor: KARLSSON, Sebastian, Deerfiled Beach, FL 33442 (US)
(86) International application number: PCT/EP2020/084054
(87) International publication number: WO 2021/148176

(56) References cited:
- US-A1- 2011 230 827
- US-A1- 2012 035 538
- US-A1- 2014 243 741

## Description

### Technical Field

The present disclosure generally relates to a container holder assembly. In particular, a container holder assembly for a medicament delivery device and a medicament delivery device comprising a container holder assembly, are provided.

### Background

Many medicament delivery devices, such as injectors, are often developed for self-administration where the user performs the injection. In some medicament delivery devices, a distal end of an injection needle protrudes into the interior of a container. This could be a drawback if the medicament reacts with the material of the injection needle when exposed for a period of time. In that respect, it is desirable to have the distal end of the injection needle outside the container until the injection is to be performed. On the other hand, this then requires that the distal end of the injection needle could be moved fairly easily into the container and also that the distal end of the injection needle is kept in a sterile environment until it is moved into the container.

Some types of medicaments can be stored for a long time and may be filled in containers, such as cartridges, syringes, ampoules, canisters or the like, containing a ready-to-use medicament in liquid state. However, some types of medicaments are a mixture of two substances, typically a medicament agent (such as lyophilized, powdered or concentrated liquid) and a diluent (such as water, dextrose solution or saline solution). These types of medicaments cannot be pre-mixed and stored for a long time because the medicament agent is unstable and will lose its effect quickly due to degradation. Hence, a user has to perform the mixing within a limited time period prior to the delivery of a dose of medicament by operating a medicament delivery device. Furthermore, some types of medicaments are sensitive to fast mixing and thus they must be mixed slowly.

In order to facilitate the mixing, a number of containers for medicament have been developed comprising at least two chambers, known as multi-chamber containers. Multi-chamber containers typically comprise a first chamber containing the medicament agent and at least one second chamber containing the diluent. These chambers may be sealed off with a stopper such that the medicament agents do not become degraded. When the medicament agent is to be mixed shortly before administering and the stopper is moved, redirecting passages are opened between the chambers. The passages allow the mixing of the medicament agent and the diluent. After sufficient mixing, the medicament is ready for delivery. Normally, delivery is performed by using injection devices wherein the multi-chamber container is arranged, more specifically in a container holder. After the mixing is performed, an injection needle having a needle sheath must be attached to the container holder and the. The step of attaching the injection needle to the container holder and thereafter removing the needle sheath is cumbersome and complicated for some users.

WO 2014076225 A1 discloses an injection needle assembly having opposite distal and proximal ends, comprising: a retainer member configured to be connectable to a medicament container; a hub configured to be coaxially displaceable within the retainer member and provided with an injection needle having proximal and distal pointed ends; and a cap member interactively connected to both the hub and to the retainer member.

WO 2009147026 A1 an injection device for manually penetrating a needle arranged to the device and automatically injecting a medicament mixture.

US 2011/230827 A1 discloses a multi-chamber injector wherein the medicaments are mixed by screwing the container holder into the casing and wherein the needle assembly is screwed into the container holder to pierce the sealing membrane of the container.

US 2014/243741 A1 discloses a multi-chamber injector wherein the container holder is screwed into the casing to mix the medicaments.

### Summary

In the present disclosure, when the term "distal direction" is used, this refers to the direction pointing away from the dose delivery site during use of the medicament delivery device. When the term "distal part/end" is used, this refers to the part/end of the delivery device, or the parts/ends of the members thereof, which under use of the medicament delivery device is/are located furthest away from the dose delivery site. Correspondingly, when the term "proximal direction" is used, this refers to the direction pointing towards the dose delivery site during use of the medicament delivery device. When the term "proximal part/end" is used, this refers to the part/end of the delivery device, or the parts/ends of the members thereof, which under use of the medicament delivery device is/are located closest to the dose delivery site.

Further, the terms "longitudinal", "longitudinally", "axially" and "axial" refer to a direction extending from the proximal end to the distal end and along the device or components thereof, typically in the direction of the longest extension of the device and/or component.

Similarly, the terms "transverse", "transversal" and "transversally" refer to a direction generally perpendicular to the longitudinal direction.

One object of the present disclosure is to provide a container holder assembly for a medicament delivery device and which container assembly is configured to receive a multi-chamber container therein, and which container holder assembly improves usability of the medicament delivery device.

A further object of the present disclosure is to provide a container holder assembly for a medicament delivery device, which container holder assembly has a simple, cheap and/or reliable design.

A still further object of the present disclosure is to provide a container holder assembly for a medicament delivery device, which container holder assembly enables a proximal end of an injection needle to be kept sterile outside a multi-chamber container prior to mixing of at least two substances in the multi-chamber container.

A still further object of the present disclosure is to provide a container holder assembly for a medicament delivery device, which container holder assembly enables multiple use of an actuator device of the medicament delivery device.

A still further object of the present disclosure is to provide a container holder assembly for a medicament delivery device, which container holder assembly solving several or all of the foregoing objects in combination.

A still further object of the present disclosure is to provide a medicament delivery device comprising a container holder assembly, which medicament delivery device solves one, several or all of the foregoing objects.

Another object of the present disclosure is to provide a container holder assembly adapted to receive a multi-chamber container therein and configured to be connectable to a medicament delivery device such that a mixing of components within the multi-chamber container is achieved by manually moving the container holder assembly into the medicament delivery device, more specifically by rotation/screwing.

According to one aspect, there is provided a container holder assembly for a medicament delivery device, the container holder assembly comprising a container holder and a needle assembly, wherein the container holder comprises a distal tubular body for receiving a multi-chamber container therein, the distal tubular body comprising an external surface and a distal body engaging structure on the external surface configured to movably engage an actuator device of the medicament delivery device in order to releasably connect the container holder to the actuator device; and a proximal tubular part comprising a proximal body engaging structure connected to the needle assembly; wherein the needle assembly comprises a hub having an injection needle with a distal needle end and a proximal needle end, the distal needle end being isolated from the multi-chamber container when the multi-chamber container is received in the distal tubular body.

The container holder assembly according enables a medicament delivery device to be delivered to a user with a pre-assembled needle assembly. The container holder assembly further enables connection to an actuator device prior to a medicament dose expulsion and disconnection from the actuator device after the medicament dose expulsion.

The injection needle may be fixedly held by the hub. The proximal needle end may extend proximally from the hub and the distal needle end may extend distally from the hub. The hub may be configured to be axially displaced relative to the container holder.

The distal tubular body may be elongated along a longitudinal axis of the container holder assembly. The distal tubular body, the proximal tubular part, the hub and/or the injection needle may be substantially coaxial, or coaxial, with the longitudinal axis.

The distal tubular body may be substantially cylindrical, or cylindrical. In this case, an interior length of the distal tubular body may be at least two times, such as at least three time, such as at least five times, an inner diameter of the distal tubular body.

The needle assembly is pre-assembled to the container holder before the multi-chamber container is received in the distal tubular body.

The distal body engaging structure may be an external thread. The external thread may be continuous or discontinuous. With external thread is meant an exterior thread. Thus, the external thread may comprise a protrusion, a recess, or both.

The distal body engaging structure may be configured to threadingly engage a housing part of the medicament delivery device. To this end, the distal body engaging structure may or may not be a thread. As a possible alternative, the distal body engaging structure may comprise one or more protrusions threadingly engaging with a thread in the housing part. However, it is also feasible that the distal body is configured to linearly engage the housing part of the medicament delivery device such that distal body can be linearly moved into the housing part without rotation.

The distal tubular body may comprise at least one engaging body structure arranged to releasably engage at least one engageable device feature in the housing part. Each of the at least one engaging body structure may be a flexible protrusion, such as a flexible tab.

The proximal body engaging structure may be an external thread for threadingly engaging the needle assembly. In addition, the proximal tubular part may comprise a proximal internal engaging structure for threadingly engaging the hub. However, it is also feasible that the proximal body engaging structure may be an external bayonet structure for engaging the needle assembly, and the proximal tubular part may comprise a proximal internal engaging structure for linearly engaging the hub without rotation.

The needle assembly may further comprise a cap. The cap may be arranged to cause axial distal displacement of the hub by manual rotation of the cap. However, it is also feasible that the cap may be arranged to cause axial distal displacement of the hub by a manual linear movement of the cap without roation.

The distal needle end may be arranged to penetrate a membrane arranged at a proximal end of the multi-chamber container by means of the axial distal displacement of the hub. The container holder assembly may further comprise a sterile barrier between the membrane and the hub. The sterile barrier may for example be provided in the proximal tubular part. Also the sterile barrier may be penetrated by the distal needle end by means of the axial distal displacement of the hub.

The needle assembly may further comprise a needle cover covering the injection needle, the needle cover comprising a distal cover engaging structure engaging the proximal body engaging structure to connect the proximal tubular part to the needle assembly. The needle cover and the cap may be an integral component. Alternatively, the cap may be provided outside the needle cover. In this case, the needle assembly may further comprise a clutch. The cap may cooperate with the needle cover via the clutch such that when the user rotates the cap in one direction, the cap and the needle cover rotate together and move proximally along the longitudinal axis. When the needle cover rotates, the hub rotates in the same direction but also moves distally along the longitudinal axis. In this way, the cap and the needle cover can be removed at the same time as, or slightly after, the distal needle end pierces the membrane of the multi-chamber container by rotation of the cap. The clutch may be a ratcheting freewheel mechanism.

The container holder assembly further comprises a tamper indication member. The tamper indication member is unbroken until the cap is removed from the container holder 18. The tamper indication member breaks to indicate tampering.

According to a further aspect, there is provided a medicament delivery device comprising a container holder assembly according to the present disclosure. The medicament delivery device may be disposable or for multiple use. The medicament delivery device may be an injection device.

The medicament delivery device may further comprise an actuator device having a housing part. In this case, the medicament delivery device may be arranged to cause mixing of at least two substances in the multi-chamber container by manually moving the container holder into the housing part, e.g. by threading engagement. The actuator device may be disposable or for multiple use.

The housing part may comprise at least two engageable device features arranged to be engaged by the engaging body structure at different axial positions of the distal tubular body relative to the housing part. Each engageable device feature may be a recess.

### Brief Description of the Drawings

Further details, advantages and aspects of the present disclosure will become apparent from the following embodiments taken in conjunction with the drawings, wherein:
- Fig. 1:: schematically represents a perspective view of a medicament delivery device comprising a container holder assembly and an actuator device;
- Fig. 2:: schematically represents a further perspective view of the medicament delivery device;
- Fig. 3:: schematically represents a cross-sectional side view of the medicament delivery device;
- Fig. 4:: schematically represents a cross-sectional and partially exploded perspective view of the container holderassembly;
- Fig. 5:: schematically represents a cross-sectional partial side view of the container holder assembly;
- Fig. 6:: schematically represents a cross-sectional and perspective partial side view of the container holder assembly;
- Fig. 7:: schematically represents a cross-sectional and partially exploded perspective view of the actuator device;
- Fig. 8:: schematically represents a cross-sectional partial side view of the actuator device; and
- Fig. 9:: schematically represents a cross-sectional and perspective partial side view of the actuator device.

### Detailed Description

In the following, a container holder assembly for a medicament delivery device and a medicament delivery device comprising a container holder assembly, will be described. The same or similar reference numerals will be used to denote the same or similar structural features.

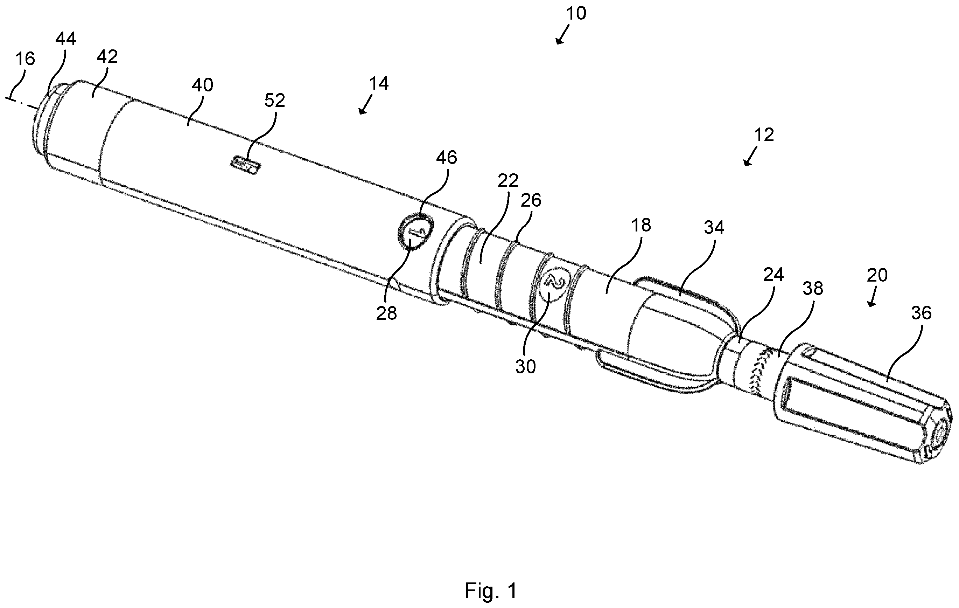

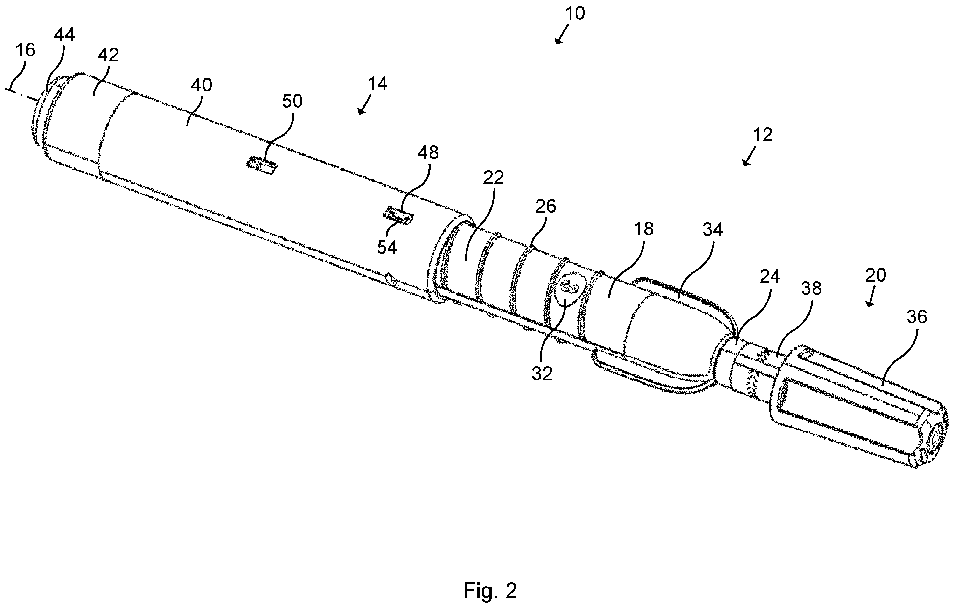

Fig. 1 schematically represents a perspective view of a medicament delivery device 10. Fig. 2 schematically represents a further perspective view of the medicament delivery device 10. With collective reference to Figs. 1 and 2, the medicament delivery device 10 of this example is an injection device. The medicament delivery device 10 comprises a container holder assembly 12 and an actuator device 14. In Figs. 1 and 2, a longitudinal axis 16 of the medicament delivery device 10 is shown.

The container holder assembly 12 comprises a container holder 18 and a needle assembly 20. The container holder 18 is connected to the needle assembly 20. Figs. 1 and 2 illustrate the medicament delivery device 10 as received by the user. Thus, the medicament delivery device 10 is delivered with the needle assembly 20 connected to the container holder 18. The actuator device 14 may be for multiple use and the container holder assembly 12 may be disposable.

The container holder 18 comprises a distal tubular body 22 and a proximal tubular part 24. A multi-chamber container (not shown) is accommodated inside the distal tubular body 22. The distal tubular body 22 comprises an external surface. On the external surface of the distal tubular body 22, a distal body engaging structure 26 is provided. The distal body engaging structure 26 is exemplified as an external thread, here a continuous external thread.

The distal tubular body 22 of this example further comprises a first indicator 28, a second indicator 30 and a third indicator 32, indicated by the numbers "1", "2" and "3", respectively. The first indicator 28 and the second indicator 30 are arranged on one side of the distal tubular body 22, and the third indicator 32 is arranged on an opposite side of the distal tubular body 22. The first indicator 28 is arranged distally of the second indicator 30 and the third indicator 32, the second indicator 30 is arranged between the first indicator 28 and the third indicator 32, and the third indicator 32 is arranged proximally of the first indicator 28 and the second indicator 30.

The container holder 18 further comprises two wings 34. In this example, the wings 34 are provided on the distal tubular body 22. The wings 34 assist the user to rotate the container holder 18.

The needle assembly 20 of this example comprises a cap 36. The cap 36 is provided around an injection needle (not shown). The cap 36 is generally cylindrical.

The container holder assembly 12 further comprises a tamper indication member 38. In Figs. 1 and 2, the tamper indication member 38 is unbroken.

When the cap 36 is removed from the container holder 18, the tamper indication member 38 breaks to indicate tampering.

The actuator device 14 comprises a housing part 40. The medicament delivery device 10 is configured to cause mixing of at least two substances in the multi-chamber container by manually moving the container holder 18 into the housing part 40.

By means of the distal body engaging structure 26, the distal tubular body 22 can movable engage the housing part 40 and the distal tubular body 22 is releasably connected to the housing part 40. Thus, the container holder 18 or the entire container holder assembly 12 can be entirely separated from the housing part 40. In this example, the distal tubular body 22 threadingly engages the housing part 40 by means of the distal body engaging structure 26.

The housing part 40 of this example is cylindrical. The container holder 18 can be at least partly received inside the housing part 40.

The actuator device 14 of this example further comprises a housing part 42. Thus, the housing part 40 is a proximal housing part and the housing part 42 is a distal housing part. In this example, the distal housing part 40 is threadingly connected to the proximal housing part 40.

The actuator device 14 of this example further comprises a button 44. The button 44 is arranged at a distal end of the actuator device 14.

The housing part 40 comprises an opening 46. As shown in Fig. 1, the first indicator 28 is aligned with the opening 46 and is visible through the opening 46. Since the first indicator 28 is aligned with the opening 46, the container holder 18 is in a first position with respect to the housing part 40. The second indicator 30 and the third indicator 32 can also be aligned with, and visible through, the opening 46 when the container holder 18 adopts a second position and a third position, respectively, along the longitudinal axis 16 with respect to the housing part 40.

The housing part 40 further comprises a first engageable device feature here exemplified as a first recess 48, a second engageable device feature here exemplified as a second recess 50, and a third engageable device feature here exemplified as a third recess 52. The distal tubular body 22 comprises an engaging body structure, here exemplified as a flexible tab 54. In the illustrated first position of the container holder 18, the flexible tab 54 releasably engages the first recess 48. The flexible tab 54 further engages the second recess 50 and the third recess 52 when the container holder 18 adopts the second position and the third position, respectively. In this way, the container holder 18 can be accurately positioned relative to the housing part 40 and the user is provided with tactile and audible feedback when the flexible tab 54 engages the respective recess 48, 50 and 52.

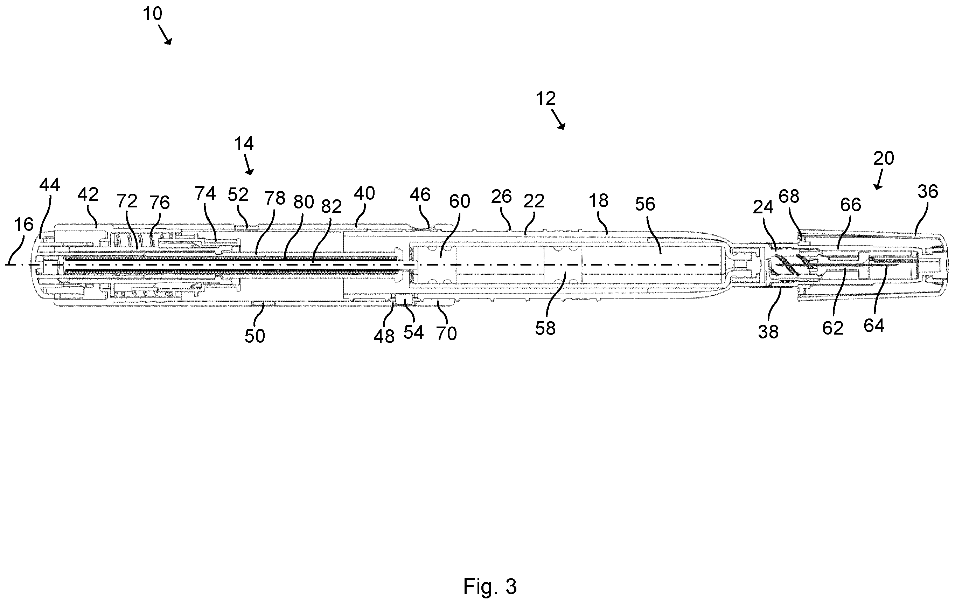

Fig. 3 schematically represents a cross-sectional side view of the medicament delivery device 10. In Fig. 3, the multi-chamber container 56 can be seen. The multi-chamber container 56 of this example is a dual-chamber container comprising a proximal stopper 58 and a distal stopper 60.

As shown in Fig. 3, the needle assembly 20 comprises, in addition to the cap 36, a hub 62 having an injection needle 64. Also the hub 62 and the injection needle 64 are concentric with respect to the longitudinal axis 16. The injection needle 64 is fixedly held by the hub 62. The needle assembly 20 is configured such that manual rotation of the cap 36 in one direction is translated to a distal movement of the hub 62 along the longitudinal axis 16.

The needle assembly 20 of this example further comprises a needle cover 66. The needle cover 66 is generally cylindrical and covers the injection needle 64. The needle cover 66 is provided radially outside the injection needle 64 and the hub 62, and radially inside the cap 36 (with respect to the longitudinal axis 16).

The needle assembly 20 of this specific example further comprises a clutch 68. The clutch 68 is provided between the cap 36 and the needle cover 66. The clutch 68 transmits rotation of the cap 36 in one direction to a rotation of the needle cover 66, the hub 62 and the injection needle 64, but does not transmit rotation of the cap 36 in an opposite direction to any rotation of the needle cover 66, the hub 62 or the injection needle 64.

As shown in Fig. 3, the distal tubular body 22 is elongated along the longitudinal axis 16. A length of the distal tubular body 22 along the longitudinal axis 16 is more than five times an interior diameter of the distal tubular body 22.

As further shown in Fig. 3, the housing part 40 comprises a housing engaging structure 70, here exemplified as a relatively short threaded portion at a proximal interior end of the housing part 40. The distal body engaging structure 26 threadingly engages the housing engaging structure 70.

Fig. 3 further shows that the actuator device 14 comprises an actuator 72, an actuator sleeve 74, a locking spring 76, a plunger rod 78, a plunger spring 80 and a guide rod 82. The plunger spring 80 surrounds the guide rod 82. The plunger rod 78 surrounds the plunger spring 80. The actuator 72, the actuator sleeve 74 and the locking spring 76 surround the plunger rod 78. In the first position of the container holder 18 illustrated in Fig. 3, the actuator sleeve 74 surrounds the actuator 72.

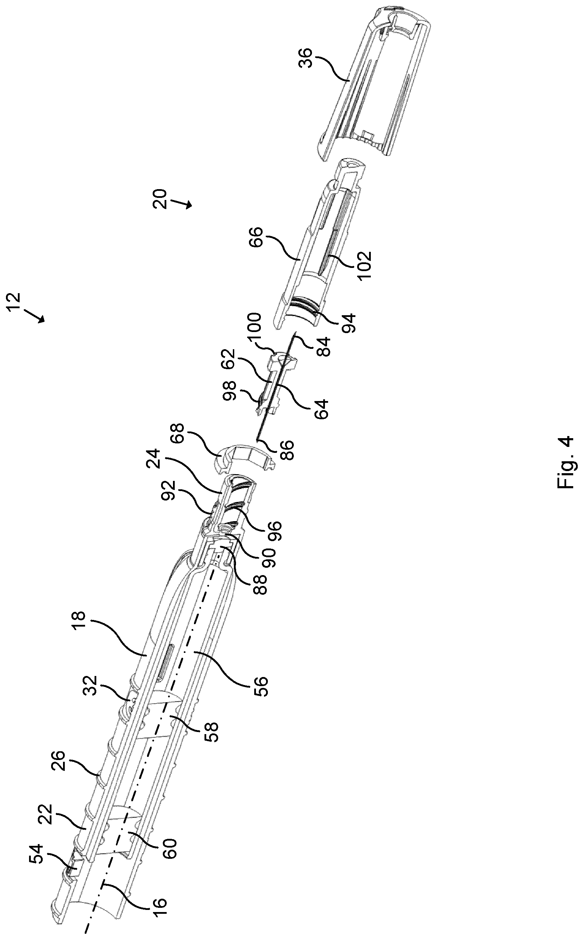

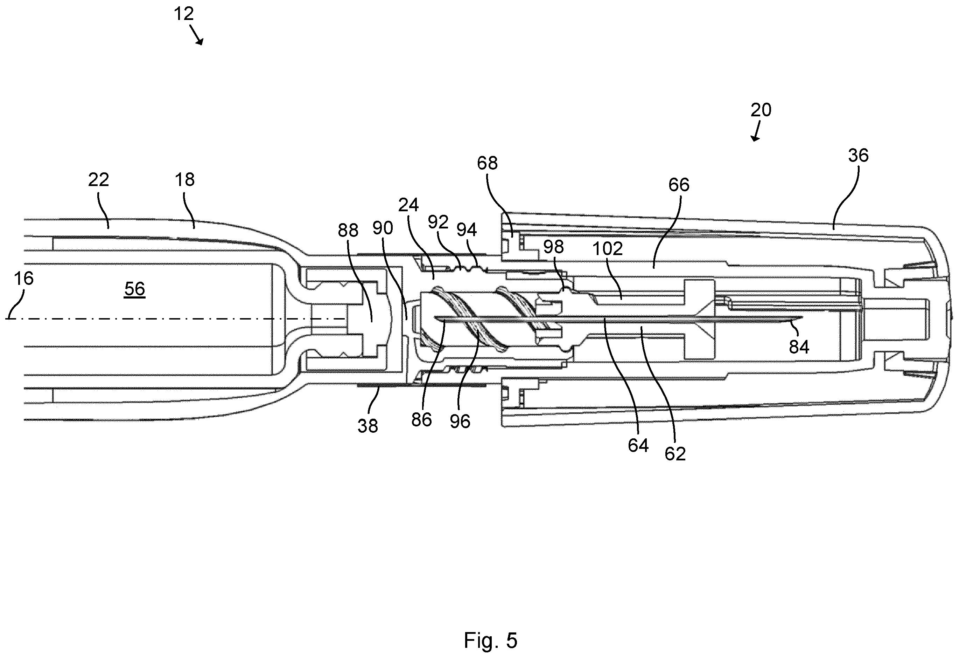

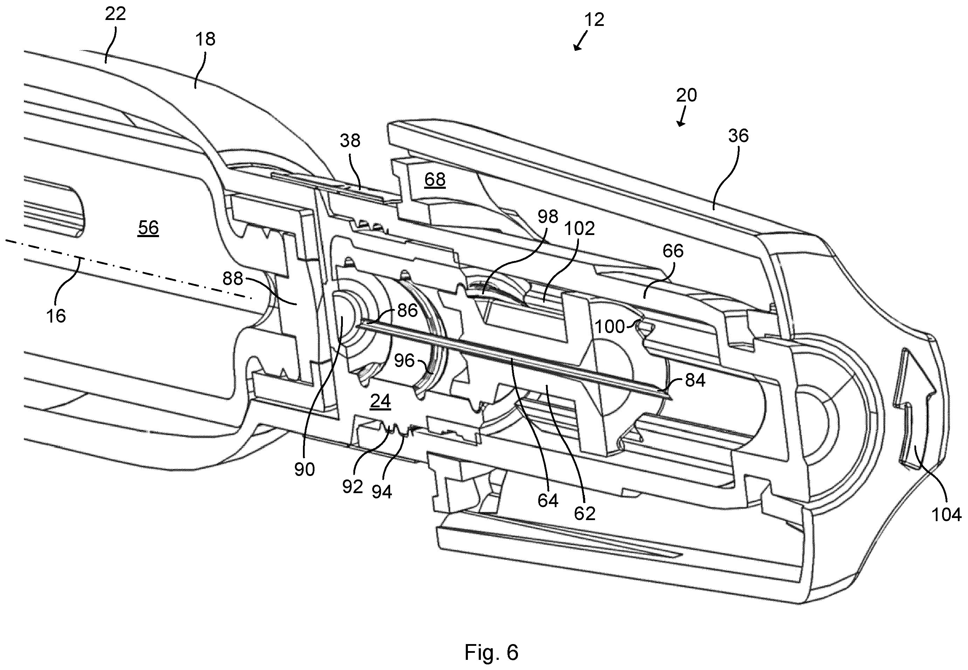

Fig. 4 schematically represents a cross-sectional and partially exploded perspective view of the container holder assembly 12, Fig. 5 schematically represents a cross-sectional partial side view of the container holder assembly 12, and Fig. 6 schematically represents a cross-sectional and perspective partial side view of the container holder assembly 12. With collective reference to Figs. 4-6, a proximal needle end 84 and a distal needle end 86 of the injection needle 64 can be seen. The proximal needle end 84 extends proximally from the hub 62 and the distal needle end 86 extends distally from the hub 62.

As shown in Figs. 4-6, the multi-chamber container 56 comprises a membrane 88 at a proximal end thereof. The multi-chamber container 56 is entirely accommodated inside the container holder 18. A major part of the multi-chamber container 56 is accommodated inside the distal tubular body 22 and a proximal end of the multi-chamber container 56 is accommodated inside the proximal tubular part 24. The multi-chamber container 56 of this example is attached to the container holder 18 by means of a snap-fit connection to the interior of the proximal tubular part 24.

The container holder 18 further comprises a sterile barrier 90, here exemplified as a transverse wall. The barrier 90 sealingly closes a proximal interior portion of the proximal tubular part 24 from a distal interior portion of the proximal tubular part 24.

In Figs. 5 and 6, it is shown that the distal needle end 86 is isolated from the multi-chamber container 56 when the multi-chamber container 56 is received in the distal tubular body 22. The distal needle end 86 is separated from the multi-chamber container 56 by means of the membrane 88 and the barrier 90.

As shown in Figs. 4-6, the proximal tubular part 24 comprises a proximal body engaging structure 92, here exemplified as a proximal external thread. The needle cover 66 comprises a distal cover engaging structure 94, here exemplified as a distal interior thread. The needle cover 66 is connected to the proximal tubular part 24 by means of a threaded engagement between the distal cover engaging structure 94 and the proximal body engaging structure 92. In this way, the needle assembly 20 is connected to the proximal tubular part 24 by means of the proximal body engaging structure 92.

The proximal tubular part 24 further comprises a proximal internal engaging structure 96, here exemplified as a proximal internal thread. The hub 62 comprises a distal external engaging structure 98, here exemplified as a distal external thread. The hub 62 is connected to the proximal tubular part 24 by means of a threaded engagement between the distal external engaging structure 98 and the proximal internal engaging structure 96.

The hub 62 further comprises proximal external grooves 100. The needle cover 66 further comprises interior ribs 102. The interior ribs 102 extend parallel with the longitudinal axis 16, in this example along more than half of a length of the needle cover 66. The proximal external grooves 100 engage a respective interior rib 102. By means of this engagement, rotation of the needle cover 66 is transmitted to a rotation of the hub 62. The engagement however enables the hub 62 to move along the longitudinal axis 16 relative to the needle cover 66.

Fig. 6 further shows that an arrow 104 is provided on the cap 36. The arrow 104 indicates a rotational direction of the cap 36 in order to remove the cap 36.

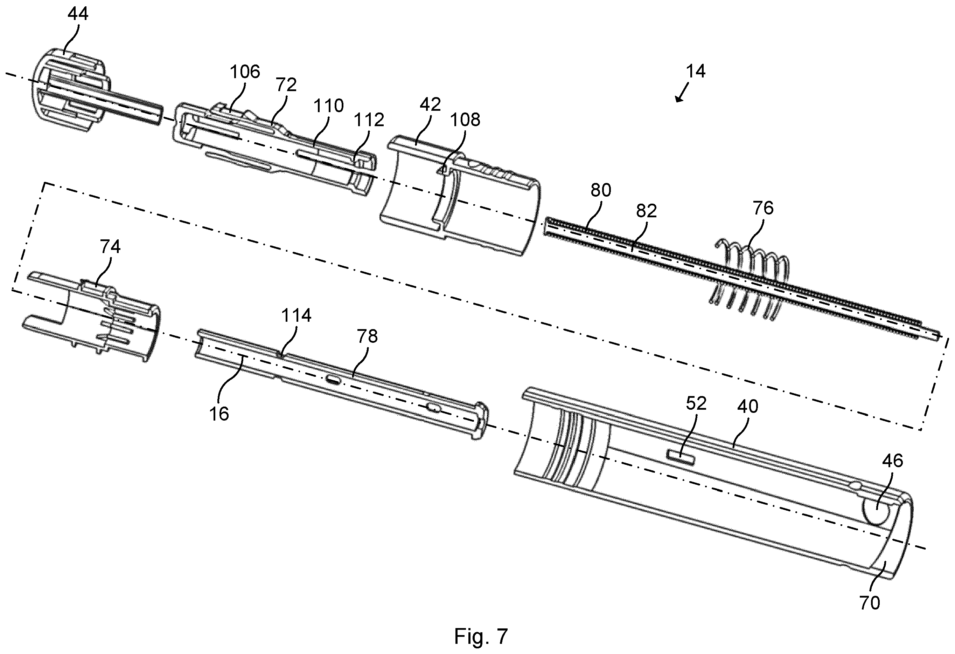

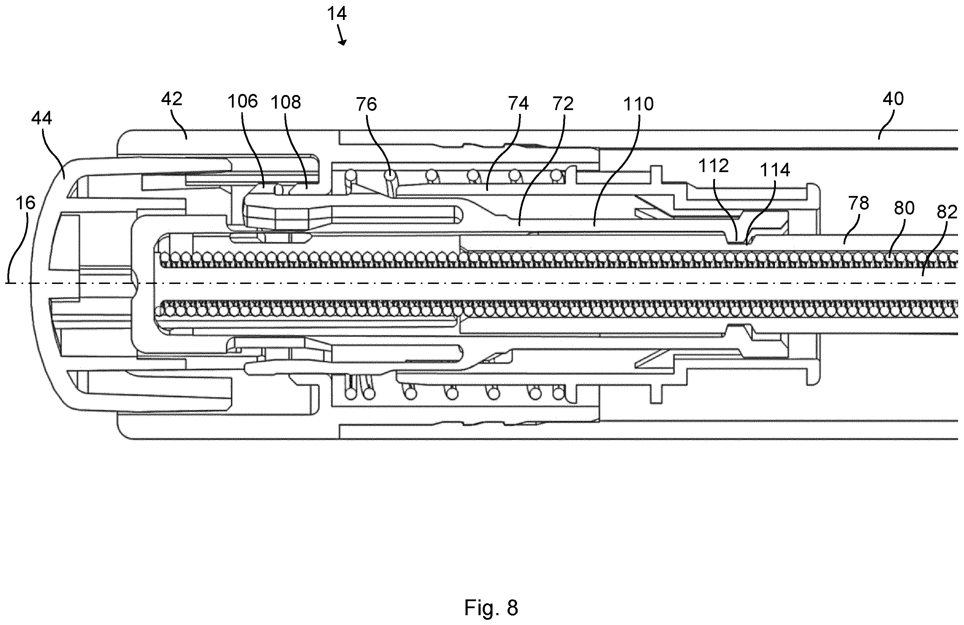

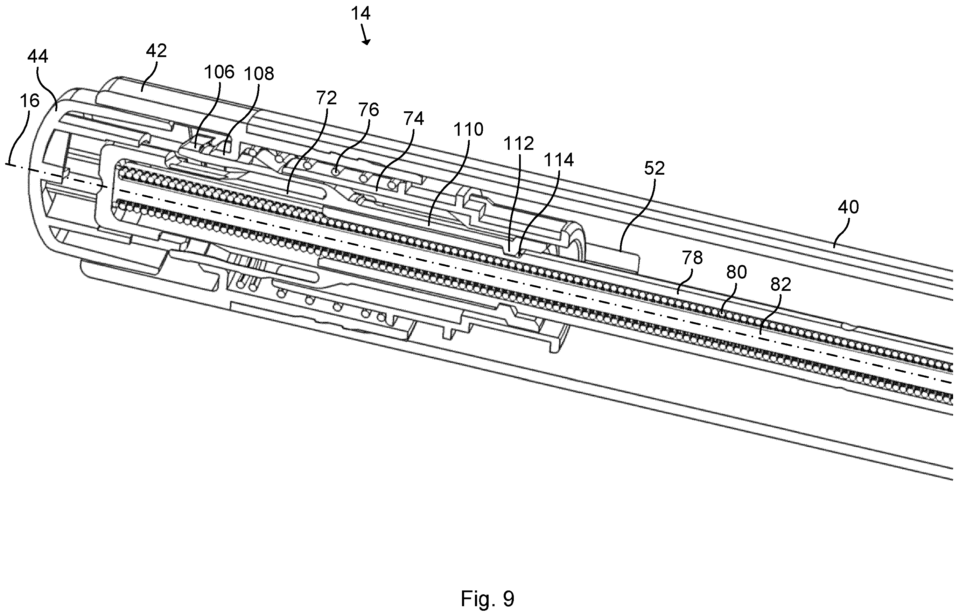

Fig. 7 schematically represents a cross-sectional and partially exploded perspective view of the actuator device 14, Fig. 8 schematically represents a cross-sectional partial side view of the actuator device 14, and Fig. 9 schematically represents a cross-sectional and perspective partial side view of the actuator device 14. With collective reference to Figs. 7-9, the actuator 72 comprises two hooks 106, here provided at a distal end of the actuator 72. The distal housing part 42 comprises a stop 108. The stop 108 protrudes radially inwards (with respect to the longitudinal axis 16). When the container holder 18 is in the first position with respect to the actuator device 14, the actuator 72 is locked since the hooks 106 engage the stop 108.

The actuator 72 of this example further comprises two flexible arms 110 projecting proximally. Each flexible arm 110 comprises a protrusion 112. The plunger rod 78 comprises a circumferential groove 114. When the container holder 18 is in the first position with respect to the actuator device 14, the protrusions 112 are seated in the groove 114 and held in the groove 114 by the actuator sleeve 74.

In the following, one exemplifying use of the medicament delivery device 10 will be described. The user may receive the medicament delivery device 10 with the container holder 18 connected to the housing part 40, the needle assembly 20 connected to the container holder 18, and a multi-chamber container 56 loaded into the distal tubular body 22.

As an alternative, the user may receive the container holder assembly 12 comprising the container holder 18 and the needle assembly 20, where the needle assembly 20 is connected to the container holder 18, and where a multi-chamber container 56 is loaded into the distal tubular body 22. In this case, the user may connect the container holder assembly 12 to the actuator device 14 by manually screwing the container holder 18 into the housing part 40 to the first position. The user knows when the housing part 40 has adopted the first position since the flexible tab 54 releasably engages the first recess 48 with a clicking sound and since the first indicator 28 (number "1") is visible through the opening 46.

In order to mix different substances contained in the multi-chamber container 56, the container holder 18 is manually rotated relatively to the housing part 40, e.g. by means of the wings 34. The container holder 18 thereby moves into the housing part 40 in a distal direction along the longitudinal axis 16. During movement of the container holder 18 from the first position towards the second position, the plunger rod 78, which is static in relation to the housing part 40, eventually shifts the distal stopper 60 proximally into the multi-chamber container 56 such that medicaments inside the multi-chamber container 56 become completely mixed.

The manual rotation continues until the container holder 18 has moved from the first position to the second position. The user knows when the housing part 40 has adopted the second position and that mixing has been completed since the flexible tab 54 releasably engages the second recess 50 with a clicking sound and since the second indicator 30 (number "2") is visible through the opening 46.

In order to prime the medicament delivery device 10, the user manually rotates the container holder 18 further relative to the housing part 40. As the container holder 18 moves from the second position towards the third position, the container holder 18 pushes the actuator sleeve 74 distally. The distal movement of the actuator sleeve 74 forces the hooks 106 inwardly away from the stop 108 to thereby unlock the actuator 72.

The manual rotation continues until the container holder 18 has moved from the second position to the third position. The user knows when the housing part 40 has adopted the third position and that priming has been completed since the flexible tab 54 releasably engages the third recess 52 with a clicking sound and since the third indicator 32 (number "3") is visible through the opening 46.

Either before or after priming, the user grabs and rotates the cap 36 in the direction illustrated by arrow 104 (Fig. 6) about the longitudinal axis 16. The rotation of the cap 36 in this direction is transmitted by the clutch 68 to a rotation in the same direction of the needle cover 66. As the needle cover 66 is rotated, the needle cover 66 moves proximally relative to the proximal tubular part 24 due to the engagement between the distal cover engaging structure 94 and the proximal body engaging structure 92. Due to this relative movement between the needle cover 66 and the proximal tubular part 24, the tamper indication member 38 breaks.

Furthermore, as the needle cover 66 is rotated, the hub 62 is rotated in the same direction due to the engagement between the interior ribs 102 and the proximal external grooves 100. As the hub 62 is rotated, the hub 62 travels distally along the longitudinal axis 16 due to the engagement between the distal external engaging structure 98 and the proximal internal engaging structure 96. As the hub 62 travels distally, the distal needle end 86 pierces the barrier 90 and the membrane 88.

When the cap 36 is rotated further, the needle cover 66 eventually disengages completely from the proximal tubular part 24. The cap 36, the clutch 68 and the needle cover 66 can then be removed to expose the proximal needle end 84.

The proximal needle end 84 of the injection needle 64 then pierces an injection site of a user. When the user pushes the button 44, the button 44 pushes the plunger rod 78 and forces the plunger rod 78 proximally. Since the protrusions 112 of the flexible arms 110 are engaged in the groove 114 of the plunger rod 78, the actuator is moved proximally by the plunger rod 78. When the flexible arms 110 pass the proximal end of the actuator sleeve 74, the plunger rod 78 is released and thereby delivers the medicament through the injection needle 64. Thus, by pressing the button 44, the medicament is automatically expelled into the injection site.

When the medicament has been expelled from the multi-chamber container 56, the plunger spring 80 forces the actuator distally against the button 44 and thereby produces an audible and tactile feedback to the user indicating injection completion. The container holder 18 may then be screwed out from the housing part 40 until the container holder 18 is disconnected from the actuator device 14. The container holder 18 and the empty multi-chamber container 56 can then be discarded and the actuator device 14 can be loaded with a new container holder assembly 12 comprising a new container holder 18 holding a full multi-chamber container 56 and being connected to a new needle assembly 20.

While the present disclosure has been described with reference to exemplary embodiments, it will be appreciated that the present invention is not limited to what has been described above. For example, it will be appreciated that the dimensions of the parts may be varied as needed. Accordingly, it is intended that the present invention may be limited only by the scope of the claims appended hereto.

## Claims

1. A medicament delivery device (10) comprising an actuator device (14) and a container holder assembly (12), the actuator device (14) comprising a housing part (40), the container holder assembly (12) comprising a container holder (18) and a needle assembly (20), wherein the container holder (18) comprises:
- a distal tubular body (22) for receiving a multi-chamber container (56) therein, the distal tubular body (22) comprising an external surface and a distal body engaging structure (26) on the external surface configured to movably engage the housing part (40) in order to releasably connect the container holder (18) to the actuator device (14), the container holder (18) being manually rotatable relatively to the housing part (40) and thereby moveable into the housing part (40) in distal direction from a first position towards at least a second position, a movement from the first position to the second position causing the medicament inside the multi-chamber container (56) to mix; and
- a proximal tubular part (24) comprising a proximal body engaging structure (92) connected to the needle assembly (20),
wherein the needle assembly (20) is pre-assembled to the container holder (18) before the multi-chamber container (56) is received in the distal tubular body (22);
wherein the needle assembly (20) comprises:
- a hub (62) having an injection needle (64) with a distal needle end (86) and a proximal needle end (84), the distal needle end (86) being isolated from the multi-chamber container (56) when the multi-chamber container (56) is received in the distal tubular body (22), the distal needle end (86) being separated from the multi-chamber container (56) by means of a membrane (88) and a barrier (90)
- a cap (36) removably attached to the container holder (18), a manual rotation of the cap (36) causing the hub (62) to move distally, thereby causing the distal needle end (86) to pierce the membrane (88) and the barrier (90).

2. A medicament delivery device (10) according to claim 1, wherein the distal tubular body (22) is elongated along a longitudinal axis (16) of the container holder assembly (12).

3. A medicament delivery device (10) according to any of the preceding claims, wherein the distal body engaging structure (26) is an external thread.

4. A medicament delivery device (10) according to claim 3, wherein the distal tubular body (22) comprises at least one engaging body structure (54) arranged to releasably engage at least one engageable device feature (48, 50, 52) in the housing part (40).

5. A medicament delivery device (10) according to claim 4, wherein each of the at least one engaging body structure (54) is a flexible protrusion.

6. A medicament delivery device (10) according to any of the preceding claims, wherein the proximal body engaging structure (92) is an external thread for threadingly engaging the needle assembly (20).

7. A medicament delivery device (10) according to any of the preceding claims, wherein the proximal tubular part (24) comprises a proximal internal engaging structure (96) for threadingly engaging the hub (62).

8. A medicament delivery device (10) according to any of the preceding claims, wherein the needle assembly (20) further comprises a needle cover (66) covering the injection needle (64), the needle cover (66) comprising a distal cover engaging structure (94) engaging the proximal body engaging structure (92) to connect the proximal tubular part (24) to the needle assembly (20).

9. A medicament delivery device (10) according to any of the preceding claims, wherein the needle assembly (20) further comprises a tamper indication member (38).

## Patentansprüche

1. Arzneimittelabgabevorrichtung (10), umfassend eine Betätigungsvorrichtung (14) und eine Behälterhalteranordnung (12), die Betätigungsvorrichtung (14) umfassend ein Gehäuseteil (40), die Behälterhalteranordnung (12) umfassend einen Behälterhalter (18) und eine Nadelanordnung (20), wobei der Behälterhalter (18) umfasst:
- einen distalen röhrenförmigen Körper (22) zum Aufnehmen eines Mehrkammerbehälters (56) darin, der distale röhrenförmige Körper (22) umfassend eine Außenoberfläche und eine distale Körpereingriffsstruktur (26) auf der Außenfläche, die konfiguriert ist, um mit dem Gehäuseteil (40) bewegbar in Eingriff zu stehen, um den Behälterhalter (18) mit der Betätigungsvorrichtung (14) lösbar zu verbinden, wobei der Behälterhalter (18) relativ zu dem Gehäuseteil (40) manuell drehbar ist und wobei dadurch in distaler Richtung von einer ersten Position zu mindestens einer zweiten Position hin in das Gehäuseteil (40) hinein bewegbar ist, wobei eine Bewegung von der ersten Position in die zweite Position das Arzneimittel im Inneren des Mehrkammerbehälters (56) veranlasst, sich zu mischen; und
- einen proximalen röhrenförmigen Teil (24), umfassend eine proximale Körpereingriffsstruktur (92), die mit der Nadelanordnung (20) verbunden ist,
wobei die Nadelanordnung (20) an dem Behälterhalter (18) vorher zusammengebaut wird, bevor der Mehrkammerbehälter (56) in dem distalen röhrenförmigen Körper (22) aufgenommen wird;
wobei die Nadelanordnung (20) umfasst:
- eine Nabe (62), die eine Injektionsnadel (64) mit einem distalen Nadelende (86) und einem proximalen Nadelende (84) aufweist, wobei das distale Nadelende (86) von dem Mehrkammerbehälter (56) isoliert ist, wenn der Mehrkammerbehälter (56) in dem distalen röhrenförmigen Körper (22) aufgenommen ist, wobei das distale Nadelende (86) von dem Mehrkammerbehälter (56) mittels einer Membran (88) und einer Barriere (90) getrennt ist
- eine Kappe (36), die an dem Behälterhalter (18) abnehmbar angebracht ist, wobei eine manuelle Drehung der Kappe (36) die Nabe (62) veranlasst, sich distal zu bewegen, wobei dadurch das distale Nadelende (86) die Membran (88) und die Barriere (90) durchsticht.

2. Arzneimittelabgabevorrichtung (10) nach Anspruch 1, wobei der distale röhrenförmige Körper (22) entlang einer Längsachse (16) der Behälterhalteranordnung (12) verlängert ist.

3. Arzneimittelabgabevorrichtung (10) nach einem der vorstehenden Ansprüche, wobei die distale Körpereingriffsstruktur (26) ein Außengewinde ist.

4. Arzneimittelabgabevorrichtung (10) nach Anspruch 3, wobei der distale röhrenförmige Körper (22) mindestens eine Eingriffskörperstruktur (54) umfasst, die angeordnet ist, um mit mindestens einem Eingriffsvorrichtungsmerkmal (48, 50, 52) in dem Gehäuseteil (40) lösbar in Eingriff zu stehen.

5. Arzneimittelabgabevorrichtung (10) nach Anspruch 4, wobei jede der mindestens einen Eingriffskörperstruktur (54) ein flexibler Vorsprung ist.

6. Arzneimittelabgabevorrichtung (10) nach einem der vorstehenden Ansprüche, wobei die proximale Körpereingriffsstruktur (92) ein Außengewinde zum in Gewindeeingriff stehen mit der Nadelanordnung (20) ist.

7. Arzneimittelabgabevorrichtung (10) nach einem der vorstehenden Ansprüche, wobei der proximale röhrenförmige Teil (24) eine proximale innere Eingriffsstruktur (96) zum in Gewindeeingriff stehen mit der Nabe (62) umfasst.

8. Arzneimittelabgabevorrichtung (10) nach einem der vorstehenden Ansprüche, wobei die Nadelanordnung (20) ferner eine Nadelabdeckung (66) umfasst, die die Injektionsnadel (64) abdeckt, die Nadelabdeckung (66) umfassend eine distale Abdeckungseingriffsstruktur (94), die mit der proximalen Körpereingriffsstruktur (92) in Eingriff steht, um den proximalen röhrenförmigen Teil (24) mit der Nadelanordnung (20) zu verbinden.

9. Arzneimittelabgabevorrichtung (10) nach einem der vorstehenden Ansprüche, wobei die Nadelanordnung (20) ferner ein Manipulationsanzeigeelement (38) umfasst.

## Revendications

1. Dispositif d'administration de médicaments (10) comprenant un dispositif d'actionnement (14) et un ensemble porte-récipient (12), le dispositif d'actionnement (14) comprenant une partie de boîtier (40), l'ensemble porte-récipient (12) comprenant un porte-récipient (18) et un ensemble d'aiguille (20), dans lequel le porte-récipient (18) comprend :
- un corps tubulaire distal (22) destiné à recevoir un récipient à compartiments multiples (56), le corps tubulaire distal (22) comprenant une surface externe et une structure d'engagement de corps distal (26) sur la surface externe configurée pour s'engager de manière mobile dans la partie de boîtier (40) afin de relier de manière amovible le support de récipient (18) au dispositif d'actionnement (14), le support de récipient (18) peut être tourné manuellement par rapport à la partie du boîtier (40) et peut ainsi être déplacé dans la partie du boîtier (40) en direction distale d'une première position vers au moins une seconde position, un mouvement de la première position vers la seconde position provoquant le mélange du médicament à l'intérieur du récipient multi-chambres (56) ; et
- une partie tubulaire proximale (24) comprenant une structure d'engagement de corps proximal (92) reliée à l'ensemble d'aiguille (20),
dans lequel l'ensemble d'aiguille (20) est pré-assemblé au porte-récipient (18) avant que le récipient multi-chambres (56) ne soit reçu dans le corps tubulaire distal (22) ;
dans lequel l'ensemble d'aiguille (20) comprend :
- un moyeu (62) comportant une aiguille d'injection (64) avec une extrémité d'aiguille distale (86) et une extrémité d'aiguille proximale (84), l'extrémité d'aiguille distale (86) étant isolée du récipient multi-chambres (56) lorsque le récipient multi-chambres (56) est reçu dans le corps tubulaire distal (22), l'extrémité d'aiguille distale (86) étant séparée du récipient multi-chambres (56) au moyen d'une membrane (88) et d'une barrière (90)
- un capuchon (36) fixé de manière amovible au support de récipient (18), une rotation manuelle du capuchon (36) entraînant le déplacement distal du moyeu (62), ce qui permet à l'extrémité distale de l'aiguille (86) de percer la membrane (88) et la barrière (90).

2. Dispositif d'administration de médicaments (10) selon la revendication 1, dans lequel le corps tubulaire distal (22) est allongé le long d'un axe longitudinal (16) de l'ensemble porte-récipient (12).

3. Dispositif d'administration de médicaments (10) selon l'une quelconque des revendications précédentes, dans lequel la structure d'engagement de corps distal (26) est un filetage externe.

4. Dispositif d'administration de médicaments (10) selon la revendication 3, dans lequel le corps tubulaire distal (22) comprend au moins une structure d'engagement de corps (54) agencée pour engager de manière libérable au moins une caractéristique de dispositif engageable (48, 50, 52) dans la partie de boîtier (40).

5. Dispositif d'administration de médicaments (10) selon la revendication 4, dans lequel chacune de l'au moins une structure d'engagement de corps (54) est une protubérance flexible.

6. Dispositif d'administration de médicaments (10) selon l'une quelconque des revendications précédentes, dans lequel la structure d'engagement de corps proximal (92) est un filetage externe pour l'engagement fileté de l'ensemble d'aiguille (20).

7. Dispositif d'administration de médicaments (10) selon l'une quelconque des revendications précédentes, dans lequel la partie tubulaire proximale (24) comprend une structure d'engagement interne proximale (96) pour l'engagement fileté du moyeu (62).

8. Dispositif d'administration de médicaments (10) selon l'une quelconque des revendications précédentes, dans lequel l'ensemble d'aiguille (20) comprend en outre un couvercle d'aiguille (66) recouvrant l'aiguille d'injection (64), le couvercle d'aiguille (66) comprenant une structure d'engagement du couvercle distal (94) s'engageant dans la structure d'engagement de corps proximal (92) pour relier la partie tubulaire proximale (24) à l'ensemble d'aiguille (20).

9. Dispositif d'administration de médicaments (10) selon l'une quelconque des revendications précédentes, dans lequel l'ensemble d'aiguille (20) comprend en outre un élément d'indication d'effraction (38)
